(19) Europäisches Patentamt
European Patent Office
Office européen des brevets

(11) Veröffentlichungsnummer: **0 152 800**
**B1**

(12) # EUROPÄISCHE PATENTSCHRIFT

(45) Veröffentlichungstag der Patentschrift: **01.04.87**

(51) Int. Cl.⁴: **C 07 C 69/88**, C 07 C 67/08

(21) Anmeldenummer: **85100740.1**

(22) Anmeldetag: **25.01.85**

(54) **Verfahren zur Herstellung von Alkylgallaten.**

(30) Priorität: **02.02.84 DE 3403575**

(43) Veröffentlichungstag der Anmeldung: **28.08.85 Patentblatt 85/35**

(45) Bekanntmachung des Hinweises auf die Patenterteilung: **01.04.87 Patentblatt 87/14**

(84) Benannte Vertragsstaaten: **BE CH DE FR GB IT LI NL**

(56) Entgegenhaltungen: **US-A-2 845 450**

(73) Patentinhaber: **BASF Aktiengesellschaft, Carl-Bosch-Strasse 38, D-6700 Ludwigshafen (DE)**

(72) Erfinder: **Rebafka, Walter, Dr., Lessingstrasse 4, D-6945 Hirschberg (DE)**
Erfinder: **Nickels, Helmut, Dr., Ginsterstrasse 15, D-6704 Mutterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 152 800 B1

## Beschreibung

Die vorliegende Erfindung betrifft ein verbessertes Verfahren zur Herstellung von Alkylgallaten der allgemeinen Formel I

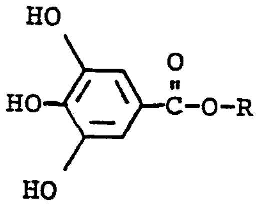

I

in der R einen $C_1$-$C_{20}$-Alkylrest bedeutet.

Gallussäure ist bekanntlich ein sehr wirksames und gleichermassen physiologisch unbedenkliches Antioxidans für Lebensmittel, Futtermittel und kosmetische Präparate, hat aber den Nachteil, dass sie sich in Fetten schlecht löst, also gerade in denjenigen Stoffen, die man in erster Linie zu stabilisieren wünscht.

Seit längerem ist es daher ebenfalls bekannt (Morris et al., J. Am. Oil Chem. Soc., 1947, Seiten 309-311), für die Stabilisierung von Fetten und fetthaltigen Zubereitungen anstelle der freien Gallussäure deren wesentlich lipophilere Alkylester zu verwenden.

Dementsprechend wurden diverse Verfahren zur Herstellung dieser Ester entwickelt, welche der zusammenfassenden Arbeit von Wijesekera et al. (J. Appl. Chem. Biotechnol., Band 22, 1972, Seiten 559-564) zu entnehmen sind. Da man bei all diesen Verfahren von reiner Gallussäure ausgeht, die ihrerseits durch Hydrolyse von tanninhaltigem Material und anschliessende recht aufwendige Isolierung gewonnen wird, ist die Herstellung der Ester insgesamt so teuer, dass diese trotz ihrer guten antioxidativen Eigenschaften bisher noch nicht in nennenswertem Ausmass verwendet wurden.

Aus der US-A-2 845 450 war es bekannt, Baumrinde mit einwertigen $C_1$-$C_4$-Alkoholen in Gegenwart saurer Katalysatoren umzusetzen, wobei man stark gefärbte undefinierte wasserlösliche Feststoffe erhält. Hierbei handelt es sich vermutlich um Derivate von Dihydroxybenzoesäureestern.

Der Erfindung lag daher die Aufgabe zugrunde, die Gallusester I, ausgehend von tanninhaltigem Material, auf wirtschaftlichere Weise zugänglich zu machen.

Demgemäss wurde ein Verfahren zur Herstellung von Alkylgallaten der allgemeinen Formel I

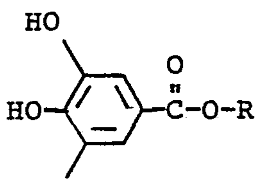

I

in der R einen $C_1$-$C_{20}$-Alkylrest bedeutet, gefunden, welches dadurch gekennzeichnet ist, dass man wasserlösliches tanninhaltiges Material mit Alkoholen R-OH (II) und starken Säuren umsetzt.

Als wasserlösliches tanninhaltiges Material eignet sich vor allem die Gruppe der hydrolysierbaren Gerbstoffe, welche wasserlösliche Ester der Gallussäure und Digallussäure mit Zuckern enthalten. Man gewinnt sie durch Wasserextraktion von Pflanzenteilen, beispielsweise des Tara, Myrobalane, Valonea, Sumach, Div-Divi und Aleppo Gallen. Diese hydrolysierbaren Gerbstoffe sind handelsüblich und liegen meist in getrockneter pulvriger Form vor, in welcher man sie im erfindungsgemässen Verfahren bevorzugt einsetzt, da sie in den Alkoholen II gut bis sehr gut löslich sind.

Das erfindungsgemässe Verfahren gelingt am besten bei Abwesenheit von Wasser, weshalb es sich empfiehlt, das in den Reaktionspartnern vorhandene sowie das entstehende Wasser kontinuierlich destillativ zu entfernen.

Als starke Säuren eignen sich beispielsweise Schwefelsäure, Chlorwasserstoff und p-Toluolsulfonsäure in möglichst wasserfreier Form. Es genügen bereits katalytische Mengen der Säure, jedoch muss man dann recht lange Reaktionszeiten in Kauf nehmen. In der Regel empfehlen sich daher höhere Konzentrationen, etwa zwischen 10 und 50 Gew.%, bezogen auf die Menge des eingesetzten Tannins.

Unter den definitionsgemässen Alkoholen II werden die unverzweigten Alkanole bevorzugt. Besondere Bedeutung hat das Verfahren für die Herstellung des n-Propyl-, n-Octyl- und n-Dodecylesters I, weil diese Substanzen bereits als Antioxidantien für Lebens- und Futtermittel eingeführt sind.

Für einen möglichst weitgehenden Umsatz des Tannins setzt man den Alkohol zweckmässigerweise in stöchiometrischen Mengen zum Gehalt der Gallussäuren in Tannin ein. In der Regel empfiehlt es sich jedoch, den Alkohol in einem bis zu etwa 30fachen molaren Überschuss über die gebundene Gallussäure zu verwenden.

Verfahrenstechnisch ist es vorteilhaft, das Tannin in dem Alkohol zu lösen, die Säure zuzugeben oder, falls diese wie HCl gasförmig ist, einzuleiten und das Gemisch unter destillativer Entfernung des Wassers zum Sieden zu erhitzen. Höhere Temperaturen als 150° C, zumal für längere Zeit, sollten jedoch wegen der thermischen Empfindlichkeit der Gallussäure vermieden werden. Mit in die Dampfphase übergehendem Alkohol kann nach Kondensation wieder in die Reaktion zurückgeführt werden, sofern er nicht oder nur schwer wasserlöslich ist.

Die Aufarbeitung des Reaktionsgemisches kann wie üblich erfolgen, indem man die Säure mit wässerigem Alkali neutralisiert, die Phase aus wasserunlöslichem Alkohol II und Ester I abtrennt, mit Wasser wäscht, trocknet, gegebenenfalls einengt und den Ester I auskristallisieren lässt. Handelt es sich um einen wasserlöslichen Alkohol, so destilliert man diesen zunächst ab, wobei sich die Phase des Esters abscheidet, der dann in einem wasserunlöslichen organischen Lösungsmittel, beispielsweise in Diethylether, Toluol, Chlorbenzol, Methylenchlorid oder einem höheren Alkohol aufgenommen und hieraus ausgefällt oder umkristallisiert wird.

Die auf diese Weise unmittelbar gewonnenen Ester I haben meistens eine Reinheit von 6-80%. Da sie als Nebenprodukte noch die ursprünglichen Naturstoff-Komponenten enthalten, können sie in dieser Form bereits als Antioxidantien für Futtermittel verwendet werden.

Höhere und höchste Reinheitsgrade erzielt man durch Umkristallisation, beispielsweise aus Chlorbenzol, Alkoholen oder Toluol oder, im Falle der niederen Ester, aus Wasser oder Methanol/Wasser-Gemischen.

Wegen der Oxidationsempfindlichkeit der Gallate empfiehlt es sich, alle Operationen unter weitgehendem Ausschluss von Luftsauerstoff, also unter Inertgasatmosphäre, vorzunehmen.

*Beispiel 1*

Herstellung von n-Propylgallat

Eine Mischung aus 500 g Tara (theoretischer Gallussäure-Gehalt ca. 1,5 mol), 2000 g (33,3 mol) n-Propanol und 50 g wasserfreier Schwefelsäure wurde 25 h unter Rückflusskühlung auf 100° C erhitzt, wobei das Reaktionswasser in Form eines Propanol/Wasser-Azeotropes laufend entfernt wurde.

Nachdem kein Wasser mehr abgespalten wurde, wurde das Gemisch mit verdünnter Natronlauge neutralisiert und destillativ vom überschüssigen Propanol befreit.

Der Rückstand wurde in einem Gemisch aus 900 ml iso-Butanol und 600 ml n-Butanol aufgenommen, wonach diese Lösung mit insgesamt 6 l Wasser gewaschen wurde.

Die organische Phase wurde eingeengt, wonach 400 g eines rohen n-Propylgallates von 60%iger Reinheit (gemäss HPLC-Analyse) in Form eines Öls erhalten wurden. Die Ausbeute, bezogen auf den Gallussäuregehalt des eingesetzten Tara und auf reinen Ester umgerechnet, betrug 75%.

Umkristallisationen aus Wasser lieferten den Ester in 60%iger Ausbeute und in einer Reinheit von 95%.

*Beispiel 2*

Herstellung von n-Octylgallat

Eine Mischung aus 30 g Tara (ca. 0,1 mol gebundene Gallussäure), 120 g (0,95 mol) n-Octanol und 3 g wasserfreier Schwefelsäure wurde unter laufender Entfernung des Reaktionswassers 7 h auf 140° C erhitzt, anschliessend mit Wasser neutral gewaschen und danach eingeengt. Hiernach wurde das rohe n-Octylgallat als Öl erhalten. Die Reinheit des Produktes betrug 65% und die Ausbeute, gerechnet als reiner Ester, 75%.

Umkristallisation aus Dichlorpropan lieferte den Ester in 65%iger Ausbeute und 95%iger Reinheit.

## Patentanspruch

Verfahren zur Herstellung von Alkylgallaten der allgemeinen Formel I

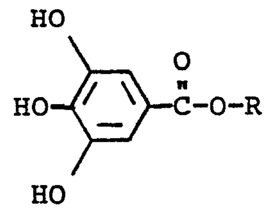

I

in der R einen $C_1$-$C_{20}$-Alkylrest bedeutet, *dadurch gekennzeichnet*, dass man wasserlösliches tanninhaltiges Material mit Alkoholen R-OH (II) und starken Säuren umsetzt.

## Claim

A process for the preparation of an alkyl gallate of the general formula I

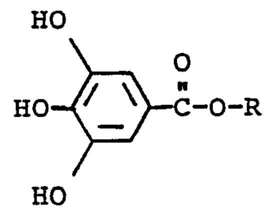

I

where R is $C_1$-$C_{20}$-alkyl, wherein water-soluble, tannin-containing material is reacted with an alcohol R-OH (II) and a strong acid.

## Revendication

Procédé pour la préparation de gallates d'alkyle de formule générale I

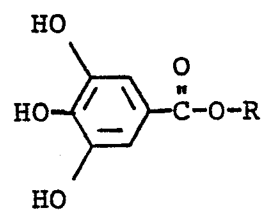

I

dans laquelle R représente un radical alkyle en $C_1$-$C_{20}$, caractérisé en ce qu'on fait réagir une matière hydrosoluble contenant des tanins avec des alcools R-OH (II) et des acides forts.